(19)

Europäisches Patentamt
European Patent Office
Office européen des brevets

(11) **EP 4 641 194 A1**

(12) **EUROPEAN PATENT APPLICATION**
published in accordance with Art. 153(4) EPC

(43) Date of publication:
**29.10.2025  Bulletin 2025/44**

(21) Application number: **23904995.0**

(22) Date of filing: **10.01.2023**

(51) International Patent Classification (IPC):
*G01N 33/44* (2006.01)   *G01N 30/88* (2006.01)
*G01N 31/16* (2006.01)   *G01N 5/04* (2006.01)
*G01N 23/223* (2006.01)   *G01N 21/3563* (2014.01)
*G16C 60/00* (2019.01)   *G16C 20/20* (2019.01)
*G16C 20/70* (2019.01)   *G16C 20/90* (2019.01)

(52) Cooperative Patent Classification (CPC):
Y02W 30/62

(86) International application number:
**PCT/CN2023/071471**

(87) International publication number:
**WO 2024/130802 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **21.12.2022   CN 202211646076**

(71) Applicant: **National Industrial Innovation Center
of Polymer
Materials Co., Ltd
Guangzhou Hi-Tech Industrial Development
Zone
Guangzhou, Guangdong 510663 (CN)**

(72) Inventors:
• **LI, Jianjun
  Guangzhou, Guangdong 510663 (CN)**

• **XIE, Xiaoqiong
  Guangzhou, Guangdong 510663 (CN)**
• **WU, Bo
  Guangzhou, Guangdong 510663 (CN)**
• **PANG, Chenghuan
  Guangzhou, Guangdong 510663 (CN)**
• **YANG, Qian
  Guangzhou, Guangdong 510663 (CN)**
• **LI, Weiling
  Guangzhou, Guangdong 510663 (CN)**
• **WU, Hao
  Guangzhou, Guangdong 510663 (CN)**
• **CHEN, Pingxu
  Guangzhou, Guangdong 510663 (CN)**
• **NING, Hongtao
  Guangzhou, Guangdong 510663 (CN)**

(74) Representative: **Meyer, Thorsten
  Meyer Patentanwaltskanzlei
  Pfarrer-Schultes-Weg 14
  89077 Ulm (DE)**

(54) **METHOD AND SYSTEM FOR IDENTIFYING HIGH-IMPACT POLYSTYRENE RECYCLED
MATERIAL**

(57)   A method and system for identifying a high-impact polystyrene (HIPS) recycled material are disclosed herein. The method includes: acquiring a HIPS sample (S101); performing sample analysis on the HIPS sample to obtain analysis result data, where the analysis result data include first analysis data representing contents of blending components, second analysis data representing a degradation degree, and third analysis data representing a low-molecular-weight compound, and the sample analysis includes acceleration factor test analysis (S102); comparing the analysis result data with feature data in a HIPS database to determine feature data corresponding to each type of the analysis result data, where the HIPS database is pre-established on the basis of HIPS plastics from various sources (S103); and identifying the HIPS sample according to feature data corresponding to the analysis result data and by a multivariate decision tree algorithm, to determine a recycled material status and a source of the HIPS sample (S104). Rapid and accurate identification of HIPS recycled materials with different content proportions and recycled material sources can be realized.

EP 4 641 194 A1

S101

acquiring a HIPS sample

S102

performing sample analysis on the HIPS sample to obtain analysis result data, wherein the analysis result data comprise first analysis data representing contents of blending components in the HIPS sample, second analysis data representing a degradation degree of the HIPS sample, and third analysis data representing a low-molecular-weight compound in the HIPS sample, and the sample analysis comprises acceleration factor test analysis

S103

comparing the analysis result data with feature data in a HIPS database to determine feature data corresponding to each type of the analysis result data, wherein the HIPS database is pre-established on the basis of HIPS plastics from various sources

S104

identifying the HIPS sample according to feature data corresponding to the analysis result data and by a multivariate decision tree algorithm, to determine a recycled material status and a source of the HIPS sample

FIG. 1

**Description**

**CROSS-REFERENCE TO RELATED APPLICATION**

This disclosure claims the benefit of Chinese patent application No. 202211646076.2, filed on December 21, 2022, which is incorporated herein by reference in its entirety.

**TECHNICAL FIELD**

The present disclosure relates to the technical field of recycled material identification, in particular to a method and system for identifying a high-impact polystyrene (HIPS) recycled material.

**BACKGROUND**

HIPS is widely used in fields such as electrical and electronic equipment, automobiles, furniture, toys, packaging, and construction. The electrical and electronic equipment is the largest application market for HIPS, accounting for 39% of the demand. To reduce carbon emissions, recycling and reusing HIPS is a preferred approach for styrene-based plastic waste. However, some food contact products have strict requirements for materials, and recycled materials cannot be used therein. Therefore, it is necessary to identify whether the product contains recycled materials. At present, PCR traceability certification, such as GRS certification and Marine plastic certification, mainly relies on trust-based document review. Currently, there is a plan to transform from trust-based document review to technical review, such that it is urgently necessary to establish a method for identifying recycled materials.

At present, for the identification of HIPS recycled materials, relevant technology includes the following steps: (1) acquiring a sample and determining whether the sample is a HIPS plastic; (2) identifying the sample by at least three of the following methods: infrared spectroscopy analysis, headspace gas chromatography analysis, electron microscopy analysis, and elemental distribution analysis; (3) determining that the sample contains HIPS plastic recycled materials if at least three of the above methods used for identification show that the sample contains HIPS plastic recycled materials. However, this technology relies on manual experience for identification, and cannot identify the source of recycled materials. For instance, if the recycled materials from electrical and electronic equipment are not completely de-halogenated, they cannot be applied to products contacted with humans, such as toys and entertainment items. Therefore, the identification of the source of recycled materials is particularly important.

**SUMMARY**

An objective of this disclosure is to overcome the shortcomings of the prior art and provide a method and system for identifying a HIPS recycled material, so as to be able to identify HIPS recycled materials with different content proportions and identify sources of the HIPS recycled materials.

To achieve the above objective, in a first aspect, provided herein is a method for identifying a HIPS recycled material, comprising:

acquiring a HIPS sample;
performing sample analysis on the HIPS sample to obtain analysis result data, wherein the analysis result data comprise first analysis data representing contents of blending components in the HIPS sample, second analysis data representing a degradation degree of the HIPS sample, and third analysis data representing a low-molecular-weight compound in the HIPS sample, and the sample analysis comprises acceleration factor test analysis;
comparing the analysis result data with feature data in a HIPS database to determine feature data corresponding to each type of the analysis result data, wherein the HIPS database is pre-established on the basis of HIPS plastics from various sources; and
identifying the HIPS sample according to feature data corresponding to the analysis result data and by a multivariate decision tree algorithm, to determine a recycled material status and a source of the HIPS sample.

In some embodiments, the HIPS database is established by taking first analysis data, second analysis data and third analysis data of a known HIPS sample as the feature data in the HIPS database, and the feature data in the HIPS database comprise feature data of a recycled material and feature data of a virgin material; the feature data of the recycled material comprise source feature data of the recycled material, and the source feature data of the recycled material represent a source of a product from which the recycled material is derived.

In some embodiments, the acceleration factor test analysis comprises:

pre-treating the HIPS sample based on a thermal-oxygen aging test or a Highly Accelerated Stress Test (HAST) to obtain a pre-treated HIPS sample;

performing a microwave extraction on the pre-treated HIPS sample, analyzing the HIPS sample after the microwave extraction by gas chromatography-mass spectrometry, to obtain a total ion current chromatogram (TIC) and a mass spectrum of the low-molecular-weight compound; and the TIC and the mass spectrum belong to the third analysis data.

In some embodiments, the HIPS plastic comprises, but not limited to, a HIPS plastic from waste electrical and electronic equipment (WEEE), a low-foamed HIPS plastic from building packaging, a HIPS plastic from food packaging, and a HIPS plastic from food tableware.

In some embodiments, the sample analysis further comprises, but not limited to, infrared spectroscopy analysis, thermogravimetric analysis, X-ray fluorescence spectroscopy analysis, differential scanning calorimetry analysis, advanced polymer chromatography analysis, a carboxyl end-group test, pyrolysis gas chromatography-mass spectrometry analysis, and physical property analysis.

In some embodiments, the advanced polymer chromatography analysis comprises:

dissolving the HIPS sample to obtain a HIPS sample solution; and
performing molecular weight analysis on the HIPS sample solution by an advanced polymer chromatograph.

In some embodiments, the pyrolysis gas chromatography-mass spectrometry analysis comprises:

performing volatile component analysis on the HIPS sample by a pyrolysis gas chromatography-mass spectrometer (Py-GCMS) with a thermal desorption temperature of 150-250 °C; and
performing chain extender analysis on the HIPS sample by the Py-GCMS with a pyrolysis temperature of 500-600°C.

In some embodiments, prior to the comparing the analysis result data with the feature data in the HIPS database to determine the feature data corresponding to each type of the analysis result data, the method further comprises:
performing dimensionality reduction on the analytical result data based on a principal component analysis method, then comparing analytical result data after dimensionality reduction with the feature data in the HIPS database.

In some embodiments, the multivariate decision tree algorithm is:

$$P = \sum_i^m a_i x_i + \sum_j^n b_j y_j + \sum_k^l c_k z_k$$
;

wherein, P represents a decision probability, m represents a feature quantity corresponding to the first analysis data, $a_i$ represents a feature variable value corresponding to the first analysis data, $x_i$ represents a feature probability corresponding to the first analysis data, $n$ represents a feature quantity corresponding to the second analysis data, $b_j$ represents a feature variable value corresponding to the second analysis data, $y_j$ represents a feature probability corresponding to the second analysis data, $l$ represents a feature quantity corresponding to the third analysis data, $c_k$ represents a feature variable value corresponding to the third analysis data, and $z_k$ represents a feature probability corresponding to the third analysis data.

In a second aspect, provided herein is a system for identifying a HIPS recycled material, comprising:

an acquisition module, configured to acquire a HIPS sample;
an analysis module, configured to perform sample analysis on the HIPS sample to obtain analysis result data, wherein the analysis result data comprise first analysis data representing contents of blending components in the HIPS sample, second analysis data representing a degradation degree of the HIPS sample, and third analysis data representing a low-molecular-weight compound in the HIPS sample, and the sample analysis comprises acceleration factor test analysis;
a comparison module, configured to compare the analysis result data with feature data in a HIPS database to determine feature data corresponding to each type of the analysis result data, wherein the HIPS database is pre-established on the basis of HIPS plastics from various sources; and
a decision module, configured to identify the HIPS sample according to feature data corresponding to the analysis result data and by a multivariate decision tree algorithm, to determine a recycled material status and a source of the HIPS sample.

Compared with the prior art, this disclosure has at least the following beneficial effects:

In this disclosure, a big data comparison between the analysis result data of a HIPS sample and the feature data in the database is conducted, to achieve the feature expression of the analysis result data, then a multivariate decision tree algorithm is utilized for analytical determination. Compared with subjective determination of the test data relying on manual labor, the influence brought by subjective factors is reduced by the method of this disclosure.

Meanwhile, the contents of blending components, the degradation degree and the low-molecular-weight compound in the HIPS sample are analyzed in this disclosure, to comprehensively consider the influence of multiple analysis result data on the identification results. Moreover, the source of HIPS recycled materials can be identified by analyzing the categories of multiple feature data and applying a decision tree algorithm, and the accuracy of the identification results is further improved.

Furthermore, based on the fact that the unsaturated C=C bond of HIPS is sensitive to oxygen, an acceleration factor test analysis method is adopted in this disclosure to amplify the difference between the HIPS recycled material and the HIPS virgin material, so as to achieve rapid and accurate identification of HIPS samples.

**BRIEF DESCRIPTION OF THE DRAWINGS**

FIG. 1 is a process diagram of the method for identifying HIPS recycled materials in an embodiment of this disclosure;
FIG. 2 shows the infrared spectrum of the sample in an embodiment of this disclosure;
FIG. 3 shows the thermogravimetric spectrum of the sample in an embodiment of this disclosure;
FIG. 4 shows the thermal analysis spectrum of the sample in an embodiment of this disclosure;
FIG. 5 shows the thermal desorption spectrum of the sample in an embodiment of this disclosure;
FIG. 6 shows the Py-GCMS spectrum of the sample in an embodiment of this disclosure;
FIG. 7 is a structure diagram of the system for identifying HIPS recycled materials in an embodiment of this disclosure.

**DETAILED DESCRIPTION**

The technical solutions of embodiments of this disclosure will be clearly and completely described below in combination with the drawings. Obviously, the described embodiments are only part of embodiments of this disclosure, not all of them. Based on the embodiments of this disclosure, all other embodiments obtained by ordinary technicians in the art without making creative efforts fall within the protection scope of this disclosure.

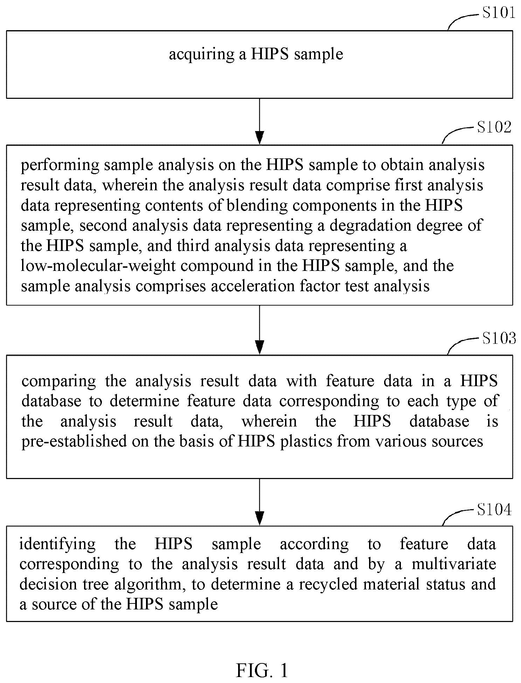

FIG. 1 is a process diagram of a method for identifying a HIPS recycled material provided by an embodiment of this disclosure. The method for identifying a HIPS recycled material in this embodiment can be applied to a system for identifying a HIPS recycled material. This system can be implemented on computer equipment, which comprises but is not limited to a tablet computer, a laptop, a desktop computer, a physical server, a cloud server, etc. The computer equipment can be connected to the instrument used in the sample analysis processes of the embodiment of this disclosure. As shown in FIG. 1, the method for identifying the HIPS recycled material of this embodiment comprises steps S101 to S104, which are described as follows:

step S101: acquiring a HIPS sample;
step S102: performing sample analysis on the HIPS sample to obtain analysis result data, wherein the analysis result data comprise first analysis data representing contents of blending components in the HIPS sample, second analysis data representing a degradation degree of the HIPS sample, and third analysis data representing a low-molecular-weight compound in the HIPS sample, and the sample analysis comprises acceleration factor test analysis; preferably, the analysis result data comprise the low-molecular-weight compound data and carboxyl index obtained after processing based on a Pressure Cooker Test (PCT) acceleration factor method;
step S103, comparing the analysis result data with feature data in a HIPS database to determine feature data corresponding to each type of the analysis result data, wherein the HIPS database is pre-established on the basis of HIPS plastics from various sources; and
step S104: identifying the HIPS sample according to feature data corresponding to the analysis result data and by a multivariate decision tree algorithm, to determine a recycled material status and a source of the HIPS sample.

In this embodiment, due to the influence of light, oxygen, heat, moisture, stress and environmental substances during the service stage of recycled materials, and the multiple processes such as sorting, cleaning, crushing, drying and granulation of waste plastics during the recycling stage, the chemical structure and properties of the materials undergo certain attenuation. The degradation of HIPS mainly occurs through the oxidation and breakage of PB chain segments in the rubber phase, which easily generates carbonyl and hydroxyl groups. Generally, a certain amount of chain extenders are added during recycling to increase the relative molecular mass of recycled-HIPS (r-HIPS) from the perspective of molecular chain repair, thereby elongating the molecular chain and further affecting the mechanical properties of the materials. Therefore, in this disclosure, contents of blending components, a degradation degree and a low-molecular-weight compound of a HIPS recycled material obtained from different sources are analyzed, a big data comparison between the analysis result data of the sample to be tested and the feature data in the database is conducted; then the sample to be tested can be determined whether it is a recycled material based on the decision tree algorithm and the comparison results, thereby identifying the recycling source of the recycled material, to achieve the identification of HIPS recycled materials and their sources.

Optionally, the HIPS database is established as follows:

Samples of HIPS recycled materials and HIPS virgin materials from different sources are collected, and subjected to sample analysis to obtain analysis data; then the HIPS database is established based on the analysis data. The analysis data of the HIPS database comprise feature data representing contents of blending components, a degradation degree and a low-molecular-weight compound (a compound with a molecular weight lower than 1000) in HIPS samples, which are used for the big data comparison in step S103. The analysis data comprise but are not limited to infrared spectroscopic data, thermogravimetric data, elemental composition data, component analysis data of a low-molecular-weight compound, and physical property data. It can be understood that for specific analysis result data, specific analysis result data can simultaneously represent multiple decision-making indicators. For instance, the elemental composition data can represent the contents of blend components in the HIPS sample as well as the low-molecular-weight compound. This will not be elaborated further here.

Optionally, the first analysis data comprise data related to the contents of the blending components between the resin and the filler in the HIPS sample. Specifically, the first analysis data comprise contents of a blending resin, a blending filler, a blending toughening agent and a blending element of the HIPS sample. For instance, the resin components detected in the HIPS recycled material include HDPE (high-density polyethylene), LDPE (low-density polyethylene), PP (polypropylene), PET (polyethylene terephthalate), polyacrylate and PVC (polyvinyl chloride) contaminants. Preferably, the element content is the content of Br, Sb, P, Ti, Ca, S, Si, Zn, CL, Mg, Al and Cd, etc.

The second analysis data comprise data related to mechanical property change, structural change (such as a glass transition temperature), and chemical change in the HIPS sample. Specifically, the second analysis data comprise but are not limited to relevant data such as an impact strength, a melt flow index, infrared carboxyl peak, a glass transition temperature, a content of carboxyl end-group, a weight-average molecular weight and a molecular weight distribution coefficient. Preferably, the second analysis data comprise the impact strength, the content of carboxyl end-group, the weight-average molecular weight and the molecular weight distribution coefficient.

The third analysis data comprise data related to a degradation product, a pollutant and an additives in the HIPS sample. Specifically, the third analysis data comprise but are not limited to the components and contents of VOCs (volatile organic compounds) and SVOCs (semi-volatile organic compounds), for example, benzene series such as 1,1,1-trichlorobenzene, toluene, chlorobenzene, cyclohexylbenzene, ethylbenzene, styrene, styrene oxides (benzaldehyde, phenol, acetophenone, etc.), dimers and trimers and oxides of styrene, as well as polycyclic benzoic acid; environmental substances such as a cleaning agent and a fragrance; degraded aldehydes, ketones and carboxylic acids; additives such as an antioxidant, a stabilizer, a flame retardant and a plasticizer. It should be noted that when an acceleration factor method (i.e., the acceleration factor test analysis) is used to analyze the third analysis data, the differences can be further amplified, which is conducive to analysis and comparison.

Optionally, the instruments used in the sample analysis processes during the establishment of the database comprise but are not limited to an infrared spectrometer, an X-ray fluorescence spectrometer (XRF), a thermogravimetric analyzer (TGA), a pyrolysis gas chromatography-mass spectrometer with thermal desorption (Py-GCMS), and a gas chromatography-mass spectrometer (GC-MS), an advanced polymer chromatograph (APC), a melt flow index tester, a differential scanning calorimeter (DSC), an impact testing machine, a PCT test oven, a strong convection thermal-oxygen aging oven, a potentiometric titrator, a microwave reactor and an ultrasonic cleaner.

Optionally, in this embodiment, the acceleration factor method is adopted to analyze the third analysis data representing the low-molecular-weight compound, to establish a database relevant to the third analysis data. The acceleration factor method comprises a thermal-oxygen aging test and a pressure cooker test (PCT). Preferably, the acceleration factor method comprises the PCT acceleration factor method.

Optionally, a strong convection thermal-oxygen aging oven is used for the thermal-oxygen aging test. The test temperature is 85-95°C, and the test sheet material has a size of $85 \times 85 \times 1$ mm or is an ordinary swatch. The PCT test is conducted by a PCT test oven. The test temperature is 85-95°C, the relative humidity in the test is 100%RH, and the test pressure is 1.0 kg/cm$^2$.

Optionally, Py-GCMS is used for the pyrolysis gas chromatography-mass spectrometry analysis. The thermal desorption temperature is 150-250 °C, the pyrolysis temperature is 600 °C, and the pyrolysis time is 0.2 min.

Optionally, a thermogravimetric analyzer is used for the thermogravimetric analysis. The temperature range of the thermogravimetric analyzer during the analysis process is 40°C-750°C, and the heating rate is 10-20 °C/min. The component analysis of both organic and inorganic substances can be conducted under a test condition with switching from nitrogen to oxygen at 600°C.

Optionally, a melt flow index tester is used for the melt flow index test. The melt flow index test is carried out at 200 °C with a loading of 5 kg. It should be noted that the melt flow index test is an analysis for representing the degradation degree of the sample.

Optionally, the differential scanning calorimetry analysis is carried out at a temperature of 30°C-300°C, with a heating rate of 10-20 °C/min, 1.5 cycles.

Optionally, a DB-5 gas chromatography column or an equivalent silica gel chromatography column (0.25 mm×30×0.25 μm) is used for GC-MS analysis.

Optionally, the HIPS sample is pre-extracted via ultrasonic extraction or microwave extraction. The microwave extraction is carried out at a temperature of 90-110°C. The extraction solvent is composed of n-hexane and isopropanol at a ratio of 50%v/v. A mass ratio of the sample to the extraction solvent is 1:10-1:30.

Optionally, HIPS plastics from various sources (HIPS recycled material samples and HIPS virgin material samples) comprise but are not limited to a HIPS plastic from waste electrical and electronic equipment (WEEE), a low-foamed HIPS plastic from building packaging, a HIPS plastic from food packaging, and a HIPS plastic from food tableware. The HIPS plastic from WEEE contains flame-retardant elements such as Br, Sb, and P, as well as a flame-retardant agent compound. The HIPS plastic from WEEE comprises a HIPS recycled material from waste plastic of a refrigerator and a HIPS recycled material from waste plastic of an air conditioner. The low-foamed HIPS plastic from building packaging contains a foaming agent. The HIPS plastic from food packaging and food tableware generally contains vegetable oils, proteins, a cleaning agent, etc. By learning the performance features of HIPS plastics from different sources, it is possible to identify the source of recycled materials for the samples to be tested based on the performance features.

Optionally, when establishing the database, the feature data that represent the performance features are analyzed based on HIPS plastics from different sources, so as to determine the feature data corresponding to the analysis result data of the sample to be tested during the big data comparison. The HIPS database is established by taking first analysis data, second analysis data and third analysis data of a known HIPS sample as the feature data in the HIPS database, and the feature data in the HIPS database comprise but are not limited to feature data of a recycled material from each source and feature data of a virgin material; the feature data of the recycled material comprise source feature data of the recycled material, and the source feature data of the recycled material represent a source of a product from which the recycled material is derived, for example, the feature data from the WEEE plastic.

Optionally, the multivariate decision tree algorithm comprises but is limited to ID3 algorithm, C4.5 algorithm, Classification and Regression Tree (CART) algorithm, and Random Forest (RF) algorithm.

It should be noted that in this disclosure, a comprehensive decision is made based on multiple decision indicators such as degradation and oxidation phenomena in recycled materials, residual low-molecular-weight compounds, and other blending components through a multivariate decision tree algorithm, which effectively avoids the one-sidedness of the identification results caused by a single decision indicator, improves the accuracy of the identification results, and makes the identification result more persuasive. Meanwhile, the decision tree is used to make a decision, which can effectively avoid human subjective influence, improve the objectivity of the identification result, and further enhance the accuracy and persuasiveness of the identification result.

In some embodiments, the acceleration factor test analysis comprises:

pre-treating the HIPS sample based on a thermal-oxygen aging test or a Highly Accelerated Stress Test (HAST) to obtain a pre-treated HIPS sample;
performing a microwave extraction on the pre-treated HIPS sample, analyzing the HIPS sample after microwave extraction by GC-MS, to obtain a total ion current chromatogram (TIC) and a mass spectrum of a low-molecular-weight compound; and the TIC and the mass spectrum belong to the third analysis data.

In this embodiment, for example, a thermal-oxygen aging test at 90 °C or a PCT method is carried out for 7 days and 3 days respectively. The microwave extraction is performed on the pre-treated sample at a temperature of 90-110°C, wherein an extraction solvent composed of n-hexane and isopropanol at a ratio of 50%v/v. The mass ratio of the sample to the extraction solvent is 1: 10-1:30. Then, the sample is analyzed by GC-MS, to obtain the TIC and the mass spectrum of a low-molecular-weight compound. The TIC and the mass spectrum of the low-molecular-weight compound are compared with the data and spectra in the HIPS database to determine the feature data corresponding to the TIC and the mass spectrum.

In some embodiments, the sample analysis also comprises but is not limited to infrared spectroscopy analysis, thermogravimetric analysis, X-ray fluorescence spectroscopy analysis, differential scanning calorimetry analysis, advanced polymer chromatography analysis, a carboxyl end-group test, pyrolysis gas chromatography-mass spectrometry analysis, and physical property analysis.

In this embodiment, the analysis result data obtained during the sample analysis belong to the first analysis data, the second analysis data or the third analysis data. For example, the elemental composition of the HIPS sample is obtained by the X-ray fluorescence spectroscopy analysis, and represents the contents of blending components or the low-molecular-weight compound in the HIPS sample. It is understandable that the sample analysis in the establishment of the database can be referred to for the analysis conditions (such as temperature and humidity, etc.) of the sample analysis in this embodiment, and will not be elaborated here.

Optionally, the advanced polymer chromatography analysis comprises:

dissolving the HIPS sample to obtain a HIPS sample solution; and
performing molecular weight analysis on the HIPS sample solution by an advanced polymer chromatograph.

In some optional embodiments, for example, the HIPS sample is dissolved in tetrahydrofuran (THF) to obtain a HIPS sample solution, and the HIPS sample solution is analyzed for the molecular weight by an advanced polymer chromatograph (APC). The APC results are compared with the data and spectra in the HIPS database to determine the feature data corresponding to the APC results. It is understandable that APC analysis also belongs to relevant feature analysis representing the low-molecular-weight compound, which further enhances the accuracy of the identification result.

Optionally, the carboxyl end-group test comprises: performing a carboxyl end-group test on the HIPS sample by a potentiometric titrator.

In some optional embodiments, the carboxyl end-group of the HIPS sample is analyzed to analyze the degradation degree, the contents of blending components and the low-molecular-weight compound of the HIPS sample. It should be noted that the carboxyl end-group test is analysis of the second analysis data.

Optionally, the pyrolysis gas chromatography-mass spectrometry analysis comprises:

performing volatile component analysis on the HIPS sample by a pyrolysis gas chromatography-mass spectrometer (Py-GCMS) with a thermal desorption temperature of 150-250°C; and
performing chain extender analysis on the HIPS sample by the Py-GCMS with a pyrolysis temperature of 500-600°C.

In some optional embodiments, for example, Py-GCMS is adopted for analysis, wherein 0.3-0.5mg of the HIPS sample is taken and analyzed for volatile component analysis by the Py-GCMS with a thermal desorption temperature of 150-250°C; then for chain extender analysis with a pyrolysis temperature of 500-600°C. The analysis result data are compared with the data and spectra in the HIPS database to determine the feature data corresponding to the analysis result data. It should be noted that the volatile component analysis belongs to an analysis of the third analysis data; and the chain extender analysis belongs to an analysis of the first analysis data.

Optionally, the infrared spectroscopy analysis comprises: focusing on the absorption peaks of carboxyl index and hydroxyl groups, other fillers or mixed plastics, and comparing the analysis result data with the data and spectra in the HIPS database to determine the feature data corresponding to the analysis result data.

Optionally, the XRF analysis comprises: sampling and cutting the HIPS sample, testing the elemental composition of the HIPS sample by XRF, quantifying the elements by a preset marking line, with particular attention paid to the flame-retardant elements Br, P, Sb and the heavy metal elements of the fillers, and comparing the analysis result data with the data and spectra in the HIPS database to determine the feature data corresponding to the analysis result data.

In some embodiments, prior to step S103, the method further comprises:
performing a dimensionality reduction on the analytical result data based on a principal component analysis method, then comparing analytical result data after the dimensionality reduction with the feature data in the HIPS database.

In this embodiment, a principal component analysis method is utilized for the dimensionality reduction, multiple indicators are converted into a few comprehensive indicators, and an algorithm is established through two-dimensional projection and threshold. The main indicators are the existence of degradation and oxidation phenomena in the recycled materials, a residual low-molecular-weight compound (an additive, a pollutant, and a degradation product of a material or a filler), and other blending polymers.

In some embodiments, the multivariate decision tree algorithm is:

$$P = \sum_i^m a_i x_i + \sum_j^n b_j y_j + \sum_k^l c_k z_k$$

wherein, P represents a decision probability, m represents a feature quantity corresponding to the first analysis data, $a_i$ represents a feature variable value corresponding to the first analysis data, $x_i$ represents a feature probability corresponding to the first analysis data, n represents a feature quantity corresponding to the second analysis data, $b_j$ represents a feature variable value corresponding to the second analysis data, $y_j$ represents a feature probability corresponding to the second analysis data, l represents a feature quantity corresponding to the third analysis data, $c_k$ represents a feature

variable value corresponding to the third analysis data, and $z_k$ represents a feature probability corresponding to the third analysis data.

In this embodiment, the feature variable value is the feature value corresponding to the feature data obtained after the comparison in the step S103, and it can be determined based on the preset correspondence between the feature data and the feature value. For example, for the bromine element (Br) in the elemental component data, its feature data are the flame retardant element interval [Br, P, S], and the corresponding feature value of this feature data is 1.

The feature probability is the feature weight determined by the decision tree algorithm during the modeling process. For example, based on HIPS plastics from multiple sources, each type of analysis result data is analyzed to obtain the feature data. By using the multivariate decision tree algorithm and based on the feature data, analysis result data are modeled until the preset convergence conditions are met (such as until no leaf nodes can be regenerated or the number of nodes reaches the preset value, etc.), and a decision tree model is obtained. The feature data output in step S103 is input into the decision tree model for decision-making, and the decision probability is output. Based on the decision probability, the decision probability can determine whether the HIPS sample belongs to a recycled material and the source of the recycled material.

As an example, rather than a limitation, the identification of whether an unknown sample is HIPS recycled material and the source of the recycled material was taken as an example. The analysis results of each sample analysis process were as follows:

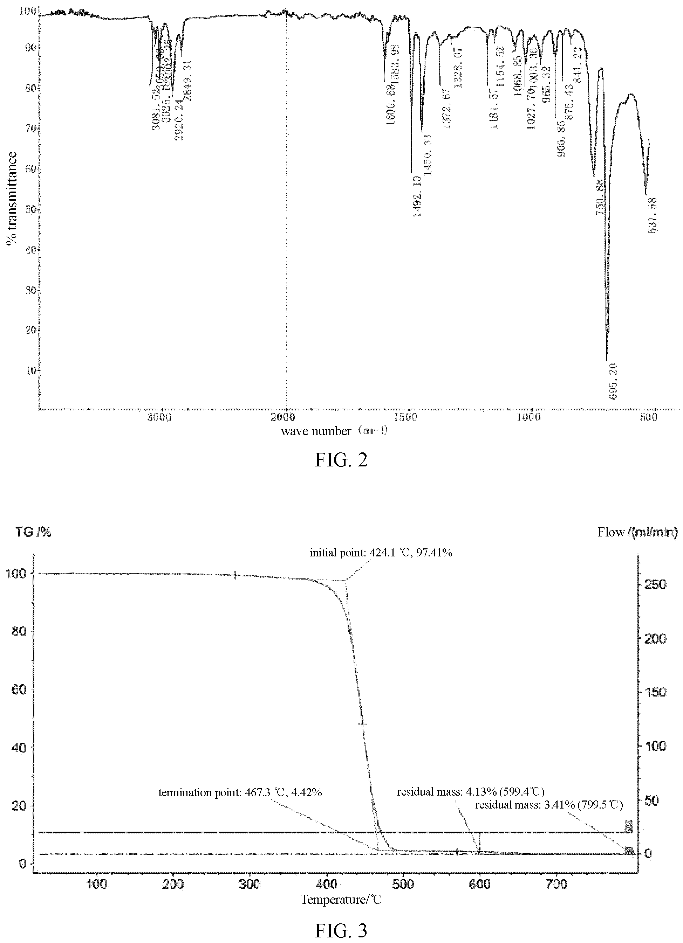

1. Infrared spectroscopy analysis: the unknown sample was analyzed by a non-destructive Attenuated Total Reflection (ATR) sampling method. The infrared spectral data of the unknown sample were shown in FIG. 2. As shown in FIG. 2, it was found that in addition to the characteristic peak of HIPS, there was also an absorption peak of the filler titanium dioxide. By comparing the infrared spectral data with the feature data in the HIPS database, it was determined that the infrared spectral data of this unknown sample belonged to the feature data of recycled materials.

2. TGA Analysis: the thermogravimetric data of the unknown sample were shown in FIG. 3. The residual mass was 3.41%, indicating that it contained 3.41 wt% ash. The ash was tested to obtain the infrared spectrum thereof, and it was found that the main components of the ash were titanium dioxide and zinc oxide. By comparing the thermogravimetric data with the feature data in the HIPS database, it was determined that the thermogravimetric data of the unknown sample belonged to the feature data boundary of modified materials and recycled materials.

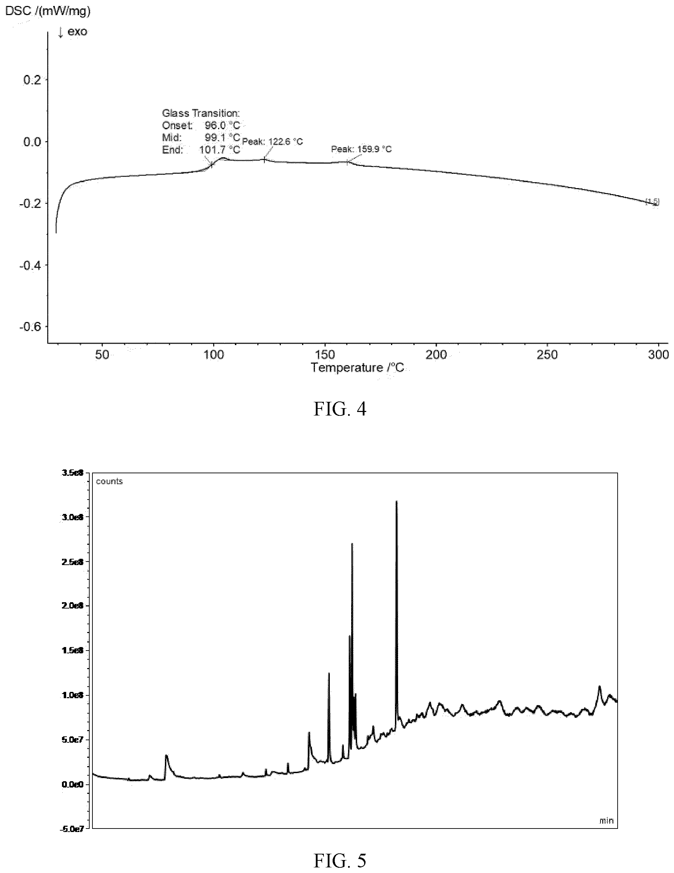

3. DSC Analysis: the DSC analysis results of the unknown sample were shown in FIG. 4. The temperature range was 30°C-300°C, the heating rate was 10°C/min, 1.5 cycles. The glass transition temperature of the sample was 99.1°C, and there were melting peaks at 122.6°C and 159.9°C. The DSC analysis results were compared with the feature data in the HIPS database, and it was determined that the unknown sample might contain trace amounts of PE (Polyethylene) and PP (Polypropylene).

4. XRF elemental analysis: the unknown sample mainly contained Ti, Zn, Br, and Sb elements, with 54.9 ppm of Br and 5.4 ppm of Sb. By comparing the XPF elemental analysis results with the feature data in the HIPS database, it was determined that the elemental composition data belonged to the feature data of recycled materials.

5. APC analysis: the unknown sample was dissolved in THF and tested for molecular weight analysis. The mobile phase was THF with a flow rate of 0.5 mL/min. Polystyrene standard samples were used for relative molecular mass calibration. The APC analysis results of the unknown samples were compared with the feature data in the HIPS database, wherein the distribution coefficient of the virgin material was between 1 and 2, and that of the recycled material was between 2.5 and 6.

| Mn (g/mol) | Mw (g/mol) | distribution coefficient |
|---|---|---|
| 9850 | 26450 | 2.68 |

6. Carboxyl end-group test: the unknown sample was subjected to the carboxyl end-group test by a potentiometric titrator, and the content of carboxyl end-groups of the unknown sample was 8 mol/t. The analytical result was compared with the feature data in the HIPS database, and it was determined that the difference was not significant.

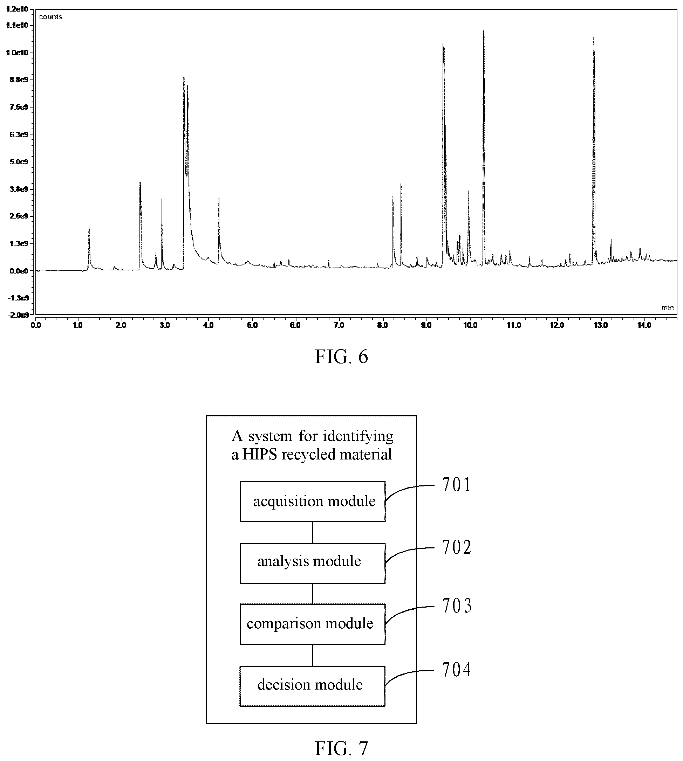

7. Pyrolysis gas chromatography-mass spectrometry analysis: Py-GCMS was adopted, 0.3-0.5mg of the unknown sample was taken, and subjected to volatile component analysis at a thermal desorption temperature of 150-250 °C, and to chain extender analysis at a pyrolysis temperature of 600°C. The analysis results of the volatile component and the chain extender were shown in FIG. 5 and FIG. 6 respectively. The two were compared with the feature data in the HIPS database. It was determined that the volatile component VOC and phenolic antioxidant components were greater than those in the virgin material components, and there were cleavage fragments of glycidyl methacrylate chain extender, indicating that the added HIPS-based macromolecule chain extender was HIPS grafted glycidyl methacrylate (HIPS-g-GMA).

8. Acceleration factor method: a PCT (with a temperature of 95°C, a humidity of 100%, 1.0 kg/cm$^2$) method was adopted and performed for 3 days. 1 g of the untreated sample and 1 g of the treated sample were taken respectively, and added to 30 mL of n-hexane/isopropanol (50%v/v). The microwave extraction was carried out at a temperature of 90°C for 30 minutes. The extract liquid was analyzed by GC-MS. The analysis results showed that there were fragments containing brominated flame retardants, as well as benzoic acid, styrene monomer, styrene dimer, styrene trimmer and 2,4-di-tert-butylphenol, etc. The analysis results were compared with the feature data in the HIPS database and it was determined that they belonged to the feature data of recycled materials from WEEE source.

It should be noted that the above analysis methods were provided as examples rather than limitations. In other embodiments, more or fewer analysis methods can be employed to analyze the analysis data representing the contents of blending components in the sample, the degradation degree of the sample, and/or the low-molecular-weight compound in the sample.

Furthermore, based on the multivariate decision tree algorithm, the feature data were used for identification. It was determined that the unknown sample belonged to a recycled material and was from WEEE waste plastics.

As another example, rather than a limitation, the unknown sample was a white particle. The sample was tested to obtain an infrared spectrum and XRF thereof, and the unknown sample was subjected to a PCT aging test, and the analysis results were compared with the feature data in the database. It was found that the unknown sample contained calcium carbonate, a foaming agent AC, degraded aldehydes, ketones, carboxylic acid substances, and VOC components. Combined with the multivariate decision tree algorithm, it was determined that the unknown sample was a foamed HIPS recycled material.

In order to carry out the method for identifying a HIPS recycled material corresponding to the embodiments, so as to achieve the corresponding functions and technical effects, FIG. 7 showed a structural block diagram of a system for identifying a HIPS recycled material, provided in an embodiment of this disclosure. For illustration, only parts related to this embodiment were shown. The system for identifying the HIPS recycled material provided in this embodiment comprised:

> an acquisition module 701, configured to acquire a HIPS sample;
> an analysis module 702, configured to perform sample analysis on the HIPS sample to obtain analysis result data, wherein the analysis result data comprised first analysis data representing contents of blending components in the HIPS sample, second analysis data representing a degradation degree of the HIPS sample, and third analysis data representing a low-molecular-weight compound in the HIPS sample, and the sample analysis comprised acceleration factor test analysis;
> a comparison module 703, configured to compare the analysis result data with feature data in a HIPS database to determine feature data corresponding to each type of the analysis result data, wherein the HIPS database was pre-established on the basis of HIPS plastics from various sources; and
> a decision module 704, configured to identify the HIPS sample according to feature data corresponding to the analysis result data and by a multivariate decision tree algorithm, to determine a recycled material status and a source of the HIPS sample.

In some embodiments, the identification system further comprised a dimension reduction module, configures to perform the dimension reduction of analysis result data based on the principal component analysis method, and to compare analysis result data after the dimension reduction with the feature data in the HIPS database.

The system for identifying the HIPS recycled material can be used to perform the method for identifying the HIPS recycled material. The options in the above method embodiments are also applicable to this embodiment and will not be elaborated here. Regarding the remaining contents of this embodiment, the contents of the above method embodiments can be referred to, and will not be elaborated in this embodiment.

The objectives, technical solutions and beneficial effects of this disclosure are further described in detail in combination with specific embodiments. It should be understood that the above descriptions are only specific embodiments of this disclosure and do not intend to limit the protection scope of this disclosure. It should be particularly pointed out that for those skilled in the art, any modifications, equivalent replacements and improvements made within the spirit and principles of this disclosure should all be covered within the protection scope of this disclosure.

**Claims**

1. A method for identifying a high-impact polystyrene (HIPS) recycled material, comprising:

> acquiring a HIPS sample;
> performing sample analysis on the HIPS sample to obtain analysis result data, wherein the analysis result data comprise first analysis data representing contents of blending components in the HIPS sample, second analysis data representing a degradation degree of the HIPS sample, and third analysis data representing a low-molecular-weight compound in the HIPS sample, and the sample analysis comprises acceleration factor test analysis;

comparing the analysis result data with feature data in a HIPS database to determine feature data corresponding to each type of the analysis result data, wherein the HIPS database is pre-established on the basis of HIPS plastics from various sources; and

identifying the HIPS sample according to feature data corresponding to the analysis result data and by a multivariate decision tree algorithm, to determine a recycled material status and a source of the HIPS sample.

2. The method for identifying a HIPS recycled material according to claim 1, **characterized in that** the HIPS database is established by taking first analysis data, second analysis data and third analysis data of a known HIPS sample as the feature data in the HIPS database, and the feature data in the HIPS database comprise feature data of a recycled material and feature data of a virgin material; the feature data of the recycled material comprise source feature data of the recycled material, and the source feature data of the recycled material represents a source of a product from which the recycled material is derived.

3. The method for identifying a HIPS recycled material according to claim 1, **characterized in that** the acceleration factor test analysis comprises:

pre-treating the HIPS sample based on a thermal-oxygen aging test or a Highly Accelerated Stress Test (HAST) to obtain a pre-treated HIPS sample;

performing a microwave extraction on the pre-treated HIPS sample, analyzing the HIPS sample after the microwave extraction by a gas chromatography-mass spectrometer, to obtain a total ion current chromatogram (TIC) and a mass spectrum of the low-molecular-weight compound; and

the TIC and the mass spectrum belong to the third analysis data.

4. The method for identifying a HIPS recycled material according to claim 1, **characterized in that** the HIPS plastic comprises a HIPS plastic from waste electrical and electronic equipment (WEEE), a low-foamed HIPS plastic from building packaging, a HIPS plastic from food packaging, and a HIPS plastic from food tableware.

5. The method for identifying a HIPS recycled material according to claim 1, **characterized in that** the sample analysis further comprises infrared spectroscopy analysis, thermogravimetric analysis, X-ray fluorescence spectroscopy analysis, differential scanning calorimetry analysis, advanced polymer chromatography analysis, a carboxyl end-group test, pyrolysis gas chromatography-mass spectrometry analysis, and physical property analysis.

6. The method for identifying a HIPS recycled material according to claim 5, **characterized in that** the advanced polymer chromatography analysis comprises:

dissolving the HIPS sample to obtain a HIPS sample solution; and
performing molecular weight analysis on the HIPS sample solution by an advanced polymer chromatograph.

7. The method for identifying a HIPS recycled material according to claim 5, **characterized in that** the pyrolysis gas chromatography-mass spectrometry analysis comprises:

performing volatile component analysis on the HIPS sample by a pyrolysis gas chromatography-mass spectrometer (Py-GCMS) with a thermal desorption temperature of 150-250°C; and

performing chain extender analysis on the HIPS sample by the Py-GCMS with a pyrolysis temperature of 500-600°C.

8. The method for identifying a HIPS recycled material according to claim 1, **characterized in that**, prior to the comparing the analysis result data with the feature data in the HIPS database to determine the feature data corresponding to each type of the analysis result data, the method further comprises:

performing a dimensionality reduction on the analytical result data based on a principal component analysis method, then comparing analytical result data after the dimensionality reduction with the feature data in the HIPS database.

9. The method for identifying a HIPS recycled material according to claim 1, **characterized in that** the multivariate decision tree algorithm is:

$$P = \sum_i^m a_i x_i + \sum_j^n b_j y_j + \sum_k^l c_k z_k$$ ;

wherein, $P$ represents a decision probability, $m$ represents a feature quantity corresponding to the first analysis data, $a_i$ represents a feature variable value corresponding to the first analysis data, $x_i$ represents a feature probability corresponding to the first analysis data, $n$ represents a feature quantity corresponding to the second analysis data, $b_j$ represents a feature variable value corresponding to the second analysis data, $y_j$ represents a feature probability corresponding to the second analysis data, $l$ represents a feature quantity corresponding to the third analysis data, $c_k$ represents a feature variable value corresponding to the third analysis data, and $z_k$ represents a feature probability corresponding to the third analysis data.

10. A system for identifying a HIPS recycled material, comprising:

an acquisition module, configured to acquire a HIPS sample;
an analysis module, configured to perform sample analysis on the HIPS sample to obtain analysis result data, wherein the analysis result data comprises first analysis data representing contents of blending components in the HIPS sample, second analysis data representing a degradation degree of the HIPS sample, and third analysis data representing a low-molecular-weight compound in the HIPS sample, and the sample analysis comprises acceleration factor test analysis;
a comparison module, configured to compare the analysis result data with feature data in a HIPS database to determine feature data corresponding to each type of the analysis result data, wherein the HIPS database is established on the basis of HIPS plastics from various sources; and
a decision module, configured to identify the HIPS sample according to feature data corresponding to the analysis result data and by a multivariate decision tree algorithm, to determine a recycled material status and a source of the HIPS sample.

```
                                                              ┌─ S101
┌──────────────────────────────────────────────────────────────┐
│                    acquiring a HIPS sample                     │
│                                                                │
└──────────────────────────────────────────────────────────────┘
                                 │
                                 ▼                            ┌─ S102
┌──────────────────────────────────────────────────────────────┐
│ performing sample analysis on the HIPS sample to obtain        │
│ analysis result data, wherein the analysis result data         │
│ comprise first analysis data representing contents of          │
│ blending components in the HIPS sample, second analysis data   │
│ representing a degradation degree of the HIPS sample, and      │
│ third analysis data representing a low-molecular-weight         │
│ compound in the HIPS sample, and the sample analysis           │
│ comprises acceleration factor test analysis                    │
└──────────────────────────────────────────────────────────────┘
                                 │
                                 ▼                            ┌─ S103
┌──────────────────────────────────────────────────────────────┐
│ comparing the analysis result data with feature data in a      │
│ HIPS database to determine feature data corresponding to       │
│ each type of the analysis result data, wherein the HIPS        │
│ database is pre-established on the basis of HIPS plastics from  │
│ various sources                                                │
└──────────────────────────────────────────────────────────────┘
                                 │
                                 ▼                            ┌─ S104
┌──────────────────────────────────────────────────────────────┐
│ identifying the HIPS sample according to feature data          │
│ corresponding to the analysis result data and by a multivariate│
│ decision tree algorithm, to determine a recycled material      │
│ status and a source of the HIPS sample                         │
└──────────────────────────────────────────────────────────────┘
```

FIG. 1

FIG. 2

FIG. 3

FIG. 4

FIG. 5

FIG. 6

FIG. 7

## INTERNATIONAL SEARCH REPORT

| International application No. |
|---|
| **PCT/CN2023/071471** |

| **A.** | **CLASSIFICATION OF SUBJECT MATTER** |
|---|---|

G01N33/44(2006.01)i; G01N30/88(2006.01)i; G01N31/16(2006.01)i; G01N5/04(2006.01)i; G01N23/223(2006.01)i; G01N21/3563(2014.01)i; G16C60/00(2019.01)i; G16C20/20(2019.01)i; G16C20/70(2019.01)i; G16C20/90(2019.01)i

According to International Patent Classification (IPC) or to both national classification and IPC

| **B.** | **FIELDS SEARCHED** |
|---|---|

Minimum documentation searched (classification system followed by classification symbols)

IPC: G01N,G16C

Documentation searched other than minimum documentation to the extent that such documents are included in the fields searched

Electronic data base consulted during the international search (name of data base and, where practicable, search terms used)

CNTXT, VEN, ENTXT, CNKI: 高抗冲聚苯乙烯, 再生料, 回收料, 加速因子, 决策树, high impact polystyrene, HIPS, reclaimed material, recycled material, accelerated factor, decision tree algorithm

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| Y | CN 111735795 A (DALIAN POLYTECHNIC UNIVERSITY) 02 October 2020 (2020-10-02) description, paragraphs 54-65, and figure 1 | 1-10 |
| Y | CN 114965973 A (ZHILI TECHNOLOGY (GUANGDONG) CO., LTD.) 30 August 2022 (2022-08-30) description, paragraphs 3-24 | 1-10 |
| A | CN 109870559 A (KINGFA SCIENCE AND TECHNOLOGY CO., LTD.) 11 June 2019 (2019-06-11) entire document | 1-10 |
| A | CN 109870561 A (KINGFA SCIENCE AND TECHNOLOGY CO., LTD.) 11 June 2019 (2019-06-11) entire document | 1-10 |
| A | CN 109883975 A (KINGFA SCIENCE AND TECHNOLOGY CO., LTD.) 14 June 2019 (2019-06-14) entire document | 1-10 |

| ☑ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 September 2023** | **20 September 2023** |

| Name and mailing address of the ISA/CN | Authorized officer |
|---|---|
| **China National Intellectual Property Administration (ISA/ CN) China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/071471** |

### C. DOCUMENTS CONSIDERED TO BE RELEVANT

| Category* | Citation of document, with indication, where appropriate, of the relevant passages | Relevant to claim No. |
| --- | --- | --- |
| A | CN 115356230 A (GUANGZHOU CUSTOMS TECHNOLOGY CENTER) 18 November 2022 (2022-11-18)<br>entire document | 1-10 |
| A | DE 19609916 A1 (MASSEN ROBERT PROF. DR. ING.) 18 September 1997 (1997-09-18)<br>entire document | 1-10 |
| A | JP 2005249624 A (JAPAN SCIENCE AND TECHNOLOGY AGENCY) 15 September 2005 (2005-09-15)<br>entire document | 1-10 |
| A | WO 2021231396 A1 (WOODS HOLE OCEANOGRAPHIC INSTITUTION) 18 November 2021 (2021-11-18)<br>entire document | 1-10 |

Form PCT/ISA/210 (second sheet) (July 2022)

**INTERNATIONAL SEARCH REPORT**

**Information on patent family members**

International application No.

**PCT/CN2023/071471**

| Patent document cited in search report | | | Publication date (day/month/year) | Patent family member(s) | Publication date (day/month/year) |
|---|---|---|---|---|---|
| CN | 111735795 | A | 02 October 2020 | None | |
| CN | 114965973 | A | 30 August 2022 | None | |
| CN | 109870559 | A | 11 June 2019 | None | |
| CN | 109870561 | A | 11 June 2019 | None | |
| CN | 109883975 | A | 14 June 2019 | None | |
| CN | 115356230 | A | 18 November 2022 | None | |
| DE | 19609916 | A1 | 18 September 1997 | None | |
| JP | 2005249624 | A | 15 September 2005 | None | |
| WO | 2021231396 | A1 | 18 November 2021 | None | |

Form PCT/ISA/210 (patent family annex) (July

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211646076